(19) Europäisches Patentamt
European Patent Office
Office européen des brevets

(11) Publication number: 0 007 716
A1

(12) # EUROPEAN PATENT APPLICATION

(21) Application number: 79301281.6

(22) Date of filing: 02.07.79

(51) Int. Cl.³: C 07 C 91/30
A 61 K 31/135, C 07 C 91/34

(30) Priority: 03.07.78 US 921666

(43) Date of publication of application:
06.02.80 Bulletin 80/3

(84) Designated Contracting States:
DE GB IT NL SE

(71) Applicant: ELI LILLY AND COMPANY
307, East McCarty Street
Indianapolis Indiana(US)

(72) Inventor: Mills, Jack
7902, Timberhill Drive
Indianapolis, Indiana 46217(US)

(72) Inventor: Schmiegel, Klaus Kurt
4507, Staughton Drive
Indianapolis, Indiana 46226(US)

(72) Inventor: Tuttle, Ronald Ralph
4540, Berkshire, Road
Indianapolis, Indiana 46226(US)

(74) Representative: McVey, Kenneth William Henry et al,
Erl Wood Manor
Windlesham Surrey, GU20 6PH(GB)

(54) **R-N-(2-phenyl-2-hydroxyethyl)-3-phenylpropyl amines, their formulations, use and preparation.**

(57) R-N-(2-Phenyl-2-hydroxyethyl)- 3-phenyl-propylamines of formula (I):

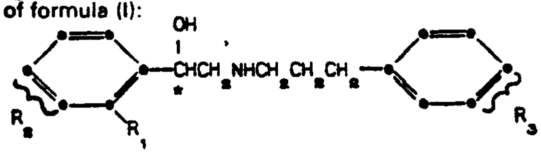

(I)

wherein:

$R_1$ is hydrogen or fluorine;

$R_2$ is hydrogen or hydroxy; provided that at least one of $R_1$ or $R_2$ is hydrogen;

$R_3$ is hydroxy;

C is an asymmetric carbon atom having the R absolute stereochemical configuration; and pharmaceutically-acceptable salts thereof, can be used in the treatment of conditions of depressed cardiac contractility or to physically condition biological systems.

EP 0 007 716 A1

# R-N-(2-PHENYL-2-HYDROXYETHYL)-3-PHENYLPROPYL AMINES, THEIR FORMULATIONS, USE AND PREPARATION

This invention relates to novel propylamine derivatives useful in the treatment of various cardiac and circulatory disorders, pharmaceutical formulations containing those derivatives and methods of preparing the novel propylamines.

Whilst numerous compounds which have inotropic properties are known, use of such compounds is severely limited due to their associated undesirable side-effects or rapid inactivation in biological systems. In many cases the treatment of congestive heart failure can only be effectively accomplished with the use of older drugs such as digitalis and allied cardiac glycosides. The main pharmacodynamic activity of digitalis resides in its ability to increase the force of myocardial contraction. Digitalis is thus widely used in the treatment of cardiac failure, and is said to be presently the fourth most frequently prescribed drug in the United States of America. Whilst digitalis and its related glycosides are widely used, they remain some of the most dangerous drugs employed. All digitalis-type preparations are toxic at high doses. Digitalis toxicity in the heart can be lethal, most of digitalis poisoning being due to the cumulative effect of maintenance doses taken over a relatively long period of time, or from use of a large dose in the treatment of severe arrhythmias.

Certain catecholamines recently have been found to exert a positive inotropic effect on heart muscle without the detrimental side effect of significant increase in heart rate. U.S. Patent No. 3,987,200 discloses a method of increasing cardiac contractility utilizing compounds such as dl-3,4-dihydroxy-N-[3-(4-hydroxyphenyl)-1-methyl-*n*-propyl]-β-phenethylamine hydrochloride, now generically referred to as dobutamine. Dobutamine is well suited for treating myocardial infarction with failure, congestive heart failure, cardiac by-pass surgery and traumatic surgery. However, like most other catechol derivatives, it is rapidly inactivated in biological systems by the action of catechol-O-methyl transferase. Dobutamine and its related drugs are thus restricted to intravenous infusion in hospitalized patients and do not lend themselves to maintenance therapy and the prophylactic treatment of heart failure.

The present invention provides a select group of phenethanolamine-type compounds which are uniquely inotropic, cause minimal undesired side effects, are not rapidly inactivated in biological systems, and are orally effective. The compounds are thus ideally suited for the treatment and maintenance of conditions of depressed cardiac contractility, and can be used prophylactically in patients suffering from heart failure.

According to the present invention there are provided compounds of formula (I):

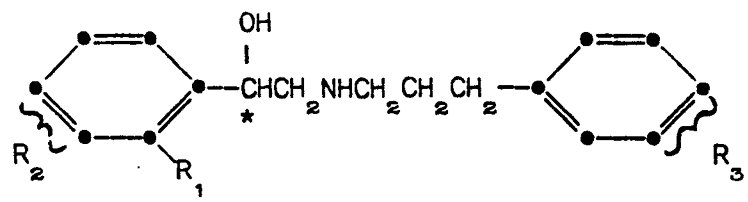

(I)

wherein:

$R_1$ is hydrogen or fluorine;

$R_2$ is hydrogen or hydroxy; provided that at least one of $R_1$ or $R_2$ is hydrogen;

$R_3$ is hydroxy;

$\underset{*}{C}$ is an asymmetric carbon atom having the R absolute stereochemical configuration; and pharmaceutically-acceptable salts thereof.

Preferred compounds of this invention are those of the above formula wherein $R_1$ is hydrogen and $R_2$ is hydroxy.

This invention additionally provides pharmaceutical formulations comprising a compound of the above formula associated with a pharmaceutically-acceptable carrier therefor.

The compounds of formula (I), or pharmaceutically-acceptable salts thereof, can be used in the treatment of conditions of depressed cardiac contractility.

The compounds of the invention can also be used to physically condition a biological system. A subject receiving such conditioning can experience

the beneficial effects of physical exercise by the administration of an effective dose of a compound of this invention and thereby obviate the actual physical work otherwise required.

The compounds of formula (I) will be named systematically herein as N-substituted 3-phenylpropylamines. For example, a compound having the above formula wherein $R_1$ is fluorine, $R_2$ is hydrogen and $R_3$ is a para-hydroxy group will be named herein as: N-[2-(2-fluorophenyl)-2-hydroxyethyl]-3-(4-hydroxyphenyl)propylamine.

As noted in the above formula (I), the compounds provided have one asymmetric carbon atom, designated in the formula as "$\overset{*}{C}$". This invention comprehends those compounds having the above formula wherein the asymmetric carbon atom has the R absolute stereochemical configuration. A complete discussion of absolute stereochemical configuration and nomenclature is presented by Cahn et al. in Experientia, Vol. XII, pp. 81-124 (1956).

The stereochemical designation of the asymmetric center in the compounds of this invention will be recited first when naming the compounds, and accordingly, the compound hereinabove named is more accurately designated R-N-[2-(2-fluorophenyl)-2-hydroxyethyl]-3-(4-hydroxyphenyl)propylamine.

The compounds provided by this invention can be prepared by any of a number of methods commonly employed in synthetic organic chemistry. A typical process for preparing compounds of this

invention comprises reacting an optically active mandelic acid or mandelic acid derivative with a 3-phenylpropylamine to form an amide, and then reducing the amide. For instance, 3-(4-hydroxyphenyl)propylamine can be acylated with a hydroxy protected mandelic acid halide such as R-2-(2-fluorophenyl)-2-acetoxyacetyl chloride to provide, after hydrolysis of the hydroxy protecting group, the corresponding amide, namely R-N-[2-(2-fluorophenyl)-2-hydroxy-1-oxoethyl]-3-(4-hydroxyphenyl)-propylamine. Such acylation reactions generally are accomplished by reacting about equimolar quantities of an acid halide or other activated acylating agent and a phenylpropylamine. The acylation is carried out in an organic solvent such as benzene, dichloromethane, dimethylformamide, or the like, and a base such as pyridine or triethylamine can be incorporated to act as an acid scavenger. The acylation typically is complete in about 6 to 36 hours, and the amide can be isolated simply by evaporation of the reaction solvent.

The amide intermediate thus mentioned can alternatively be prepared by simply coupling a mandelic acid with a phenylpropylamine utilizing any of the known peptide coupling agents. Such agents include N,N'-dicyclohexylcarbodiimide, carbonyldiimidazole, N-ethoxycarbonyl-2-ethoxy-1,2-dihydroquinoline and the like. For instance, a mandelic acid such as R-mandelic acid can be stirred with about equimolar quantities of a phenylpropylamine such as 3-(3-hydroxyphenyl)propylamine and a coupling agent such as N-ethoxycarbonyl-2-ethoxy-1,2-dihydroquinoline. The coupling reactions are best conducted in solvents such as benzene, dimethylformamide, dichloromethane, or the like, and accelerators such as 1-hydroxybenzotriazole can be incorporated if desired. The reaction typically is complete within about 2 to about 48 hours when carried out at a temperature of about -30°C. to about 150°C. The product amide is isolated by simply filtering the reaction mixture and washing it with acid and base, and then evaporating the reaction solvent.

The amide which thus is produced next is reduced to provide a compound of this invention. Reduction of an amide carbonyl can be accomplished by routine methods, for instance by reaction of the amide with diborane or other suitable reducing agent. Such reduction normally is carried out in an ethereal solvent such as diethyl ether or tetrahydrofuran, and generally is complete in about 6 to about 72 hours when carried out from about 0°C. to about 60°C. For example, an amide such as R-N-[2-4-hydroxyphenyl)-2-hydroxy-1-oxo-ethyl]-3-(4-hydroxyphenyl)propylamine can be reacted with excess diborane in tetrahydrofuran for about 30 hours at 50°C. to effect complete reduction of the amide carbonyl group. Any excess diborane remaining in the reaction mixture can be decomposed by diluting the reaction mixture with an alcohol such as methanol or with water. Removal of the reaction

solvent then provides the optically active amine of this invention, which can be further purified if desired by normal methods, including chromatography, crystallization, acid addition, salt formation and the like.

Such a process will normally proceed via a protected amine of formula (II):

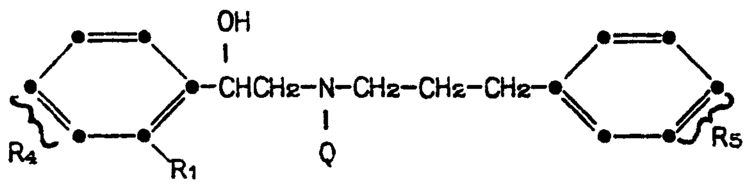

(II)

wherein $R_1$ is a previously defined, $R_4$ and $R_5$ are identical with $R_2$ and $R_3$ or can independently represent -OQ, where Q represents a protecting group, preferably benzyl, the protecting groups being hydrogenolyzed off using conventional reagents known to effect such a process, for example by catalytic hydrogenation at a temperature from 15 to 110°C. in a polar organic solvent for example methanol. The compounds of formula (II) are novel and are provided in one aspect of the invention.

Still another synthetic procedure for preparing the compounds of this invention comprises reacting a phenylpropylamine such as 3-(3-hydroxyphenyl)propylamine with a benzoyl methyl halide such as 2-fluorobenzoylmethyl bromide. The alkylation affords the corresponding N-benzoylmethyl-phenylpropylamine, for example, N-(2-fluorobenzoylmethyl)-3-(3-hydroxyphenyl)propylamine. The benzoyl carbonyl group next is reduced to provide a compound of

this invention. Such reduction can be accomplished by reaction with any of a number of common reducing agents, including hydrogenation over a catalyst such as palladium on carbon or Raney nickel. Such reduction clearly gives a mixture of optical isomers and separation of such optical isomers can be achieved by routine methods including chromatography and resolution. Alternatively, the mixture of such optical isomers can be utilized as such according to this invention since the S isomers of compounds having the above formula are substantially devoid of biological activity.

The amide which is reduced may be represented by the structural formula (III)

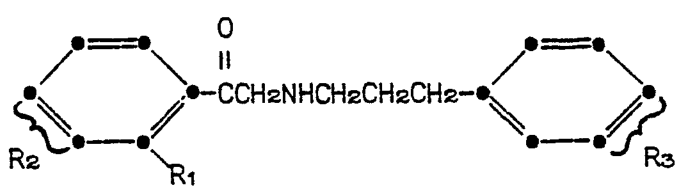

(III)

where $R_1$, $R_2$ and $R_3$ are as defined previously. These compounds of formula (III) are novel and are provided in a further aspect of the invention.

A preferred group of compounds provided by this invention are those having the above general formula in which both of the phenyl rings bear a hydroxyl group. When preparing such compounds it may be desirable to derivatize the hydroxyl groups so as to protect them during chemical reactions, thus avoiding any interference which might be caused by free hydroxyl groups. Phenolic as well as

alkyl hydroxyl groups can be protected with any of a number of commonly used hydroxyl protecting groups. The use of such groups is described in detail by E. Haslam in Protective Groups In Organic Chemistry, J.F.W. McOmie, Ed., Plenum Press, New York, N.Y. 1973, Chapter 3. Examples of commonly used protecting groups include ether forming groups such as benzyl, methyl, methoxymethyl, trimethylsilyl; ester forming groups such as acetate, benzoate, 2,2-dichloroacetate, 2,2,2-trichloroacetate, phenylsulfonate, and related groups.

A typical example of the preparation of a compound of this invention wherein both $R_2$ and $R_3$ are hydroxy and suitable protecting groups are utilized involves as a first step the preparation of the protected reactants. For instance, R-*para*-hydroxymandelic acid can be protected by reaction with benzyl chloride to provide R-2-(4-benzyloxyphenyl)-2-hydroxyacetic acid. The 2-hydroxy group also can be protected if desired, for instance by reaction with dichloroacetyl chloride or other suitable protecting group, thus providing, as an example, R-2-(4-benzyloxyphenyl)-2-dichloroacetoxyacetic acid. Such protected mandelic acid derivative is coupled to a suitably protected phenylpropylamine, for instance 3-(4-benzyloxyphenyl)-propylamine or N-benzyl-3-(4-benzyloxyphenyl)-propylamine. The amide which is produced next is reduced as hereinabove described to provide, in the instant case, a multiply protected amine. Protecting groups such as 2,2-dichloroacetyl readily are removed by reaction with a base such as 5 N sodium hydroxide, and protecting groups such as benzyl generally are removed by hydrogenation in the presence of catalysts such as Raney nickel.

Since the compounds of this invention are amines they are basic in nature by virtue of the amino group. As such, the compounds are capable of forming salts with any of a number of inorganic and organic acids. An additional embodiment of this invention encompasses the pharmaceutically acceptable salts formed by reaction of the amines of the above general formula with acids. The term "pharmaceutically acceptable salt" as used herein refers to those amine acid addition salts which can be utilized in a biological system without imparting undesirable side effects attributable to the particular acid utilized to form the salt. While the identity of the particular acid used to form a pharmaceutically acceptable salt is not critical, the salt which is formed utilizing such acid must be pharmaceutically acceptable. Acids commonly utilized to form salts according to this invention include mineral acids such as hydrochloric, hydrobromic, phosphoric, sulfuric, perchloric, nitric, and related acids. Routinely used organic acids include acetic, butyric, citric, maleic, succinic, fumaric, lactic, methanesulfonic, p-toluenesulfonic, and similar organic acids.

Like most amine salts, the pharmaceutically acceptable acid addition salts comprehended by this invention are characteristically highly crystalline solids, and thus lend themselves to ready purification by recrystallization from common solvents such as ethanol, methanol, acetone, water, and the like. As will be described more fully hereinbelow, the salts of this invention are often preferred over the free amine bases as medicaments since they are easily formulated for convenient oral or parenteral administration. It will of course be recognized that an acid addition salt of this invention can readily be converted to the corresponding free amine simply by reaction with a base such as aqueous sodium hydroxide or potassium hydroxide.

Exemplary of the compounds contemplated by this invention are the following:

R-N-(2-phenyl-2-hydroxyethyl)-3-(3-hydroxyphenyl)propylamine;

R-N-(2-phenyl-2-hydroxyethyl)-3-(4-hydroxyphenyl)propylamine;

R-N-(2-phenyl-2-hydroxyethyl)-3-(3-hydroxyphenyl)propylaminium chloride;

R-N-(2-phenyl-2-hydroxyethyl)-3-(4-hydroxyphenyl)propylaminium acetate;

R-N-[2-(2-fluorophenyl)-2-hydroxyethyl]-3-(3-hydroxyphenyl)propylamine;

R-N-[2-(2-fluorophenyl)-2-hydroxyethyl]-3-(4-hydroxyphenyl)propylaminium bromide;

R-N-[2-(3-hydroxyphenyl)-2-hydroxyethyl]-3-(3-hydroxyphenyl)propylamine;

R-N-[2-(3-hydroxyphenyl)-2-hydroxyethyl]-3-(4-hydroxyphenyl)propylamine;

R-N-[2-(3-hydroxyphenyl)-2-hydroxyethyl]-3-(4-hydroxyphenyl)propylaminium para-toluenesulfonate;

R-N-[2-(4-hydroxyphenyl)-2-hydroxyethyl]-3-(3-hydroxyphenyl)propylamine;

R-N-[2-(4-hydroxyphenyl)-2-hydroxyethyl]-3-(3-hydroxyphenyl)propylaminium isobutyrate; and

R-N-[2-(4-hydroxyphenyl)-2-hydroxyethyl]-3-(4-hydroxyphenyl)propylaminium oxalate.

As already pointed out, pharmaceutical formulations containing one or more compounds of this invention in combination with a suitable pharmaceutical diluent or carried therefor are provided as a further embodiment of the invention. Such formulations are useful in the treatment of heart failures due to conditions of depressed cardiac contractility and can also be used to physically condition a subject who cannot or will not physically condition by exercising.

The compounds of this invention can be formulated by any of a number of commonly used methods for convenient administration orally, intravenously, intramuscularly, dermally, sublingually, rectally, via aerosols, buccal seals, and the like. A phenethanolamine of the invention, as the free base or acid addition salt, can be admixed

with commonly used diluents and carriers, including starch powder, sucrose, dextrose, cellulose fiber, sorbitol, mannitol, liquid paraffin, calcium silicate, silica, polyvinylpyrrolidone, cocoa butter, methyl cellulose, methyl hydroxybenzoate, ethyl lactate, sorbitan trioleate and related excipients and carriers. The formulations of this invention can additionally contain more than one active ingredient of this invention, as well as the pharmacologically less inotropically active S-isomers. The formulations normally will contain from about 1 to about 30 percent by weight of active ingredient. The formulated phenethanolamines can be encapsulated by an ingestible carrier in the form of a capsule, sachet, cachet, buccal seal, or the like, thus providing for convenient oral administration. Alternatively the formulations can be molded into tablets in unit dosage form. For intravenous administration, the formulated phenethanolamines are best dissolved in a suitable solvent such as isotonic saline or glucose or sterile water.

An additional aspect of this invention includes a method of treating heart failure due to conditions of depressed cardiac contractility. According to such method of treatment, a dose effective for increasing cardiac contractility is administered to a subject suffering from heart failure due to a condition of depressed cardiac contractility and inadequate ventricular function or inadequate pumping function or to a subject suspected of developing heart failure due to conditions of inadequate cardiac pumping function. The compounds can be formulated for convenient oral, topical, rectal or parenteral administration, and typically are administered in the form of a nontoxic pharmaceutically acceptable acid addition salt. The particular route of administration will vary according to the particular condition and subject to be treated. In cases of severe heart failure due to acutely depressed cardiac contractility, it may be desirable to administer a phenethanolamine of this invention intravenously until the pumping function is improved, at which time the patient can be maintained by intramuscular or oral administration. Additionally, the compounds of this invention can be formulated for administration via buccal seals, by sublingual lozenges, by rectal suppositories, by metered aerosols and by dermal application. These latter named routes of administration are particularly preferred in the prophylactic treatment of inadequate ventricular function for which the compounds of this invention are well suited.

For intravenous administration according to the method of treatment of this invention, an optically active phenethanolamine of the above general formula is administered at the rate of about 0.01 to about 10 mcg./kg./min. until the contractile force of the heart muscle is restored. The infusion at such rate is continued until the subject shows signs of relief, at which time the rate of infusion can be decreased.

Maintenance therapy can be accomplished if desired by the intramuscular injection of a compound of this invention in the amount of from about 10 to about 50 mcg./kg. of body weight at intervals of from about one to four times per day, or as required by the severity of the cardiac condition being treated as well as the tolerance displayed by the patient. Maintenance therapy also is conveniently accomplished by oral administration of capsules containing sufficient quantity of a compound of this invention so that the effective dose is from about 5 to about 200 mcg./kg. of body weight. Since the acid addition salts of the phenethanolamine bases of the above formula are characteristically highly soluble in water, it is preferred that oral administration is carried out utilizing formulations containing a phenethanolamine in the form of a pharmaceutically acceptable acid addition salt. For example, a particularly preferred method of treatment according to this invention comprises administering a dose effective for increasing the pumping force of a heart muscle of R-N-[2-(4-hydroxyphenyl)-2-hydroxyethyl]-3-(4-hydroxyphenyl)propylaminium chloride. Such compound is ideally administered orally, sublingually, rectally or dermally at a dose of from about 20 to about 150 mcg./kg. to a subject suffering from heart failure, or prophylactically to a subject suspected of developing heart failure due to conditions of inadequate heart pumping function.

The compounds provided by this invention have been evaluated for their inotropic potency in anesthetized dogs and in conscious dogs with implanted cardiovascular transducers. The heart rate, cardiac output, systolic pressure and the derivative of the left ventricular pressure, which is the index of cardiac contractility, were measured. The compounds provided by this invention proved to be strongly inotropic, direct acting with immediate onset and adequate duration of action, and have displayed no alpha receptor activity. That the compounds of this invention are potent inotropic agents is demonstrated by the biological data presented in Table I.

Column 1 lists the compounds evaluated according to the test. Each of the compounds evaluated was administered intravenously to four dogs with induced myocardial infarction. Column 2 records the contractile potency of each compound as the dose in mcg./kg. of body weight required to effect a fifty percent increase in contractility ($ED_{50}$). Column 3 presents the increase in heart rate observed at the $ED_{50}$ dose. Column 4 records the change in blood pressure attributable to the administered drug.

TABLE I

| Compound evaluated | Contractile potency (mcg./kg.) $ED_{50}$ | Heart rate increase (beats/min.) $ED_{50}$ | Blood pressure change (mm/Hg) $ED_{50}$ |
|---|---|---|---|
| R-N-[2-(3-hydroxyphenyl-2-hydroxyethyl]-3-(4-hydroxyphenyl)propylamine | 5 | 12 | +1 |
| R-N-[2-(3-hydroxyphenyl)-2-hydroxyethyl]-3-(3-hydroxyphenyl)propylamine | 1 | 14 | +7 |
| R-N-[2-(4-hydroxyphenyl)-2-hydroxyethyl]-3-(3-hydroxyphenyl)propylamine | 1 | 16 | -4 |
| R-N-[2-(4-hydroxyphenyl)-2-hydroxyethyl]-3-(4-hydroxyphenyl)propylamine | 4 | 10 | +4 |

Because the compounds comprehended by this invention have unique biological effects, it has been found that they also can be used to physically condition a subject, that is to say that they effect an increase in the oxidative capacity of skeletal muscle. It is now widely recognized that physical training benefits people with occlusive peripheral or coronary artery disease. The result of such physical exercising is that skeletal muscle adapts in such a way that it can make better use of available blood flow. From the physiological standpoint, physical training causes an increase in the number or size, or both, of mitochondria, thereby enhancing the ability of skeletal muscle to utilize oxygen. This in turn permits the muscle to do more work on the same or lesser amount of blood flow. Therefore, a restriction of blood flow caused by a diseased artery supplying the skeletal muscle becomes less of a handicap. Moreover, since less blood flow is required by skeletal muscle, the demands on the heart are decreased so that the heart's need for blood flow is also reduced. Additionally, a restriction of blood flow caused by obstructive coronary artery disease in the heart becomes less of a handicap.

A further benefit occuring with physical training is a decrease in heart rate when the body is under stress from exercise. Heart rate determines the number of times per minute that the heart develops blood pressure, and naturally a decrease in heart rate lowers cardiac blood flow. This is not a

problem, however, since the training also decreases the requirement of peripheral tissue for blood flow, thus reducing cardiac output requirements. Surprisingly, the compounds provided by this invention cause the same effects observed with physical training, namely increased oxidative capacity of skeletal muscle, reduced cardiac blood flow requirements, as well as significant reduction in heart rate during exercise, a reduction in cardiac work and coronary blood flow. The compounds provided herein thus represent a significant advance in the art, since many of the people who need physical training the most are unable to exercise because of physical handicaps, arthritis, or peripheral vascular disease or existing heart disease such that exercise may either precipitate or exacerbate symptoms of heart failure. By chronic treatment with a compound of this invention, the effects of physical conditioning can be realized without the physical exertion, thus resulting in an increased efficiency for the entire cardiovascular system.

A further aspect of this invention therefore is a method for physically conditioning a subject, i.e. a method for increasing oxidative capacity of skeletal muscle, by administering an effective dose of a phenethanolamine of this invention. According to such method, a compound of this invention is administered to a subject at a dose of about 5 mcg./kg. to about 200 mcg./kg. Preferably, the compound is initially administered at a

relatively low dose, for instance from about 5 to about 10 mcg./kg., at intervals of once or twice each day for about one or two weeks. The effective dose then is increased to a dose of about 10 to about 20 mcg./kg. once or twice each day for about one or two weeks. The drug dose is then again increased at regular intervals until the maximum degree of physical conditioning is experienced without an adverse increase in heart rate. For example, such dose build up procedure can be carried out by administering effective amounts of a compound such as R-N-(2-phenyl-2-hydroxyethyl)-3-(4-hydroxyphenyl)-propylaminium bromide. Such administration is preferably carried out orally at an initial dose of about 10 mcg./kg. Alternatively, such compound can be administered dermally in the form of a cream at an initial dose of about 15 to about 20 mcg./kg. The dosage, by whichever route selected, is gradually increased until the desired degree of conditioning of the biological system is established. The oral administration of a compound such as R-N-(2-phenyl-2-hydroxyethyl)-3-(4-hydroxyphenyl)propylaminium bromide may reach, for instance, an upper limit of about 60 to about 80 mcg./kg. given about once or twice each day. Such dosage will be continued as needed to maintain the desired effects of physical conditioning. Such maintenance therapy may be accomplished by the administration of the drug in the amount of about 60 to about 80 mcg./kg. from about one to three times per week as required.

The following detailed Examples illustrate the preparation and use of certain compounds comprehended by this invention. The Examples are not intended to include all aspects of the invention and should not be so construed.

Example 1

R-N-[2-(3-Hydroxyphenyl)-2-hydroxyethyl]-3-(3-hydroxyphenyl)propylamine

A solution of R-2-(3-benzyloxyphenyl)-2-dichloroacetoxyacetyl chloride was reacted with an equimolar quantity of N-benzyl-3-(3-benzyloxyphenyl)-propylamine to provide the corresponding amide, namely R-N-[2-(3-benzyloxyphenyl)-2-dichloroacetoxy-1-oxoethyl]-N-benzyl-3-(3-benzyloxyphenyl)propylamine as an oil. A solution of 15.2 g. of the amide so formed in 150 ml. of tetrahydrofuran was added dropwise over thirty minutes to a stirred solution of 105 ml. of 1 N diborane in tetrahydrofuran. Following complete addition, the reaction mixture was heated to reflux and stirred for fifteen and one-half hours. The reaction mixture next was cooled to 40°C. and diluted by the dropwise addition of 30 ml. of water to decompose any excess diborane reducing agent. The reaction mixture was made acidic by the addition of 65 ml. of 3 N hydrochloric acid, and then heated to reflux and stirred for an additional two hours. The acid mixture then was cooled to 44°C. and made alkaline by the addition of 105 ml. of 5 N sodium hydroxide, and then stirred for one hour at

about 50°C. After cooling the mixture to room temperature, the product was extracted therefrom into ethyl acetate. The organic extract was washed with water, dried and the solvent was removed by evaporation under reduced pressure to provide 12.3 g. of R-N-[2-(3-benzyloxyphenyl)-2-hydroxyethyl]-N-benzyl-3-(3-benzyloxyphenyl)propylamine as an oil. $[\alpha]_D$ -23.6° (MeOH).

A solution of 5.7 g. of the amine so formed in 115 ml. of methanol containing 1.0 g. of five percent palladium on carbon was stirred at 60°C. for 12 hours under a hydrogen pressure of 50 psi. The reaction mixture then was filtered and the filtrate was concentrated to dryness by evaporation of the solvent. The residue was dissolved in ethyl acetate and added to a solution of fumaric acid in diethyl ether. The amine acid addition salt precipitated out of solution and was collected by filtration and dried (2.5 g.). The solid so formed was suspended in fresh ethyl acetate and diluted with aqueous potassium carbonate, which thus provided the free amine which was soluble in the ethyl acetate. The organic layer was separated, dried, and the solvent was removed by evaporation to provide 1.6 g. of R-N-[2-(3-hydroxyphenyl)-2-hydroxyethyl]-3-(3-hydroxyphenyl)propylamine. $[\alpha]_D^{27°C}$ -7.4°, $[\alpha]_{365}^{27°C}$ -10.5° (MeOH)

Analysis calc. for $C_{17}H_{21}NO_3$

Theory: C, 71.06; H, 7.37; N, 4.87.

Found: C, 71.09; H, 7.20; N, 5.07.

## Example 2

Following the general procedure set forth in Example 1, 14.7 g. of R-N-[2-(3-benzyloxyphenyl)-2-dichloroacetoxy-1-oxoethyl]-N-benzyl-3-(4-benzyloxyphenyl)propylamine was reduced by reaction with 105 ml. of 1 N diborane in tetrahydrofuran to provide, after workup with sodium hydroxide, 11.7 g. of R-N-[2-(3-benzyloxyphenyl)-2-hydroxyethyl]-N-benzyl-3-(4-benzyloxyphenyl)propylamine. The latter compound was subjected to hydrogenation to remove the benzyl protecting groups to provide, after isolation and purification, 2.0 g. of R-N-[2-(3-hydroxyphenyl)-2-hydroxyethyl]-3-(4-hydroxyphenyl)propylamine. $[\alpha]_D^{27°C}$ -15.5°, $[\alpha]_{365}^{27°C}$ -29.5° (MeOH)

Analysis calc. for $C_{17}H_{21}NO_3$

Theory: C, 71.06; H, 7.37; N, 4.87.

Found: C, 71.22; H, 7.54; N, 4.86.

## Example 3

N-(2-Phenyl-2-hydroxyethyl)-3-(4-hydroxyphenyl)propylamine

A solution of 50 g. of benzoylmethyl bromide in 200 ml. of diethyl ether was added dropwise over one hour to a stirred solution of 83 g. of 3-(4-methoxyphenyl)propylamine in 500 ml. of diethyl ether. The reaction mixture was stirred for forty-eight hours at room temperature and then filtered. The ethereal reaction mixture was extracted several times with ten percent aqueous hydrobromic acid. The

acidic extracts were combined and heated at 100°C. for twelve hours. The precipitate which had formed was collected by filtration, triturated three times with 50 ml. portions of acetone, and recrystallized from ethanol, water and ethyl acetate to provide 30.8 g. of N-(2-phenyl-2-oxoethyl)-3-(4-methoxyphenyl)propylaminium bromide. M.P. 199-200°C.

Analysis calc. for $C_{18}H_{22}NO_2Br$
Theory: C, 59.35; H, 6.09; N, 3.85.
Found: C, 59.21; H, 6.45; N, 3.89.

A solution of 25.2 g. of the product thus formed in 250 ml. of glacial acetic acid containing 100 ml. of 48 percent hydrobromic acid was heated to reflux and stirred for four hours. The acidic reaction mixture was cooled to room temperature and concentrated to a volume of about 50 ml. whereupon a crystalline product precipitated. The precipitate was collected by filtration and recrystallized three times from water, ethanol and diethyl ether, thus affording 17.8 g. of N-(2-phenyl-2-oxoethyl)-3-(4-hydroxyphenyl)propylaminium bromide. M.P. 176-179°C.

Analysis calc. for $C_{17}H_{20}NO_2Br$
Theory: C, 58.30; H, 5.76; N, 4.00.
Found: C, 58.09; H, 5.99; N, 3.96.

A solution of 15.8 g. of the amine salt thus formed in ninety-five percent ethanol in water was hydrogenated in the presence of 1.5 g. of five-

percent palladium on carbon for twelve hours at 25°C. under a hydrogen pressure of 60 psi. The reaction mixture was filtered and the solvent was evaporated from the filtrate to provide a solid residue. The solid was crystallized from ethyl acetate and diethyl ether, and then recrystallized twice from ethanol and ethyl acetate to provide 14.5 g. of dl-N-(2-phenyl-2-hydroxyethyl)-3-(4-hydroxyphenyl)propylaminium bromide. M.P. 151-152°C.

Analysis calc. for $C_{17}H_{22}NO_3Br$

Theory: C, 57.96; H, 6.30; N, 3.98.

Found: C, 57.71; H, 6.35; N, 3.98.

The mixture of isomers thus produced can be separated by normal resolution techniques but preferably is used as the mixture since the inotropically less active S-isomer displays few if any undesirable side effects.

## Example 4

Following the general procedure set forth in Example 3, 4-benzyloxybenzoyl methyl bromide was reacted with 3-(3-methoxyphenyl)propylamine to provide N-[2-(4-benzyloxyphenyl)-2-oxoethyl]-3-(3-methoxyphenyl)propylaminium bromide. M.P. 208-208.5°C. Reaction of the latter compound with glacial acetic acid and hydrobromic acid effected de-protection of the phenolic hydroxyl groups and provided N-[2-(4-hydroxyphenyl)-2-oxoethyl]-3-(3-hydroxyphenyl)propylaminium bromide. M.P. 158-160°C. Catalytic hydrogenation then provided,

after purification by crystallization, dl-N-[2-(4-hydroxyphenyl)-2-hydroxyethyl]-3-(3-hydroxyphenyl)propylaminium bromide. M.P. 163.5-164.5°C.

Analysis calc. for $C_{17}H_{22}NO_3Br$

Theory: C, 55.45; H, 6.02; N, 3.80.

Found: C, 55.30; H, 6.06; N, 4.07

Example 5

A typical formulation suited for oral administration as comprehended by this invention contains the following ingredients.

| | |
|---|---|
| R-N-[2-(3-hydroxyphenyl)-2-hydroxyethyl]-3-(4-hydroxyphenyl)propylaminium chloride | 10 mg. |
| Stearic acid | 10 mg. |
| Dextrose | 480 mg. |
| Total | 500 mg. |

The ingredients are admixed with a corn starch paste and the mixture is compressed into tablets. The tablets are administered to a subject in need of increased cardiac contractility at the rate of about 1 to about 2 tablets per day.

Example 6

Preparation for Oral Suspension

| | |
|---|---|
| R-N-[2-(4-hydroxyphenyl)-2-hydroxyethyl]-3-(4-hydroxyphenyl)propylaminium oxalate | 25 mg. |
| Sorbitol solution (70% N.F.) | 50 ml. |
| Sucrose | 10 mg. |
| Cherry flavor | 10 mg. |
| Distilled water q.s. ad | 100 mg. |

The sorbitol solution is added to 40 ml. of distilled water and the active ingredient is dissolved therein. The sucrose and flavoring are added and dissolved. The volume is adjusted to 100 ml. with distilled water. Each ml. of syrup contains about 250 mcg. of active ingredient.

Example 7

Preparation of Buccal Seal for Sublingual Administration

| | |
|---|---|
| R-N-[2-(3-hydroxyphenyl)-2-hydroxyethyl]-3-(3-hydroxyphenyl)propylaminium *para*-toluenesulfonate | 10 mg. |
| Lactose | 150 mg. |
| Ethanol | 10 mg. |

The active drug and lactose are dissolved in the ethanol and the solution is added to the cavity of a buccal seal made of gums such as guar

gum, tragacanth, gum acacia and the like. The ethanol is then removed by evaporation to leave a uniform mixture of the drug and lactose deposited as a solid in the buccal seal. The buccal seal then is orally administered and attaches to the inner membrane of the mouth and thereby provides for the gradual release of the drug.

## CLAIMS

1. A compound of formula (I)

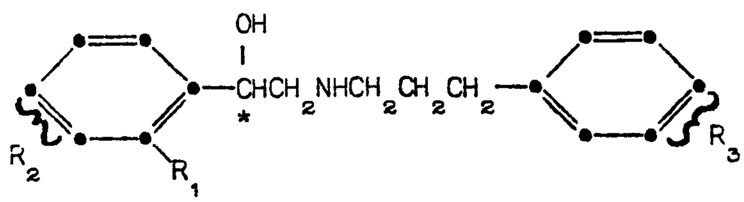

(I)

wherein:

$R_1$ is hydrogen or fluorine;

$R_2$ is hydrogen or hydroxy; provided that at least one of $R_1$ or $R_2$ is hydrogen;

$R_3$ is hydroxy;

$C_*$ is an asymmetric carbon atom having the R absolute stereochemical configuration; or a pharmaceutically-acceptable salt thereof.

2. A compound of formula (I) as claimed in claim 1, wherein $R_2$ is hydroxy.

3. A compound of formula (I) as claimed in claim 2 selected from the group comprising

R-N-[2-(3-hydroxyphenyl)-2-hydroxyethyl]-3-(3-hydroxyphenyl)propylamine,

R-N-[2-(3-hydroxyphenyl)-2-hydroxyethyl]-3-(4-hydroxyphenyl)propylamine,

R-N-[2-(4-hydroxyphenyl)-2-hydroxyethyl]-3-(3-hydroxyphenyl)propylamine, and

R-N-[2-(4-hydroxyphenyl)-2-hydroxyethyl]-3-(4-hydroxyphenyl)propylamine.

4. A compound of formula (I) as claimed in any one of claims 1 to 3, associated with its corresponding S-isomer.

5. A pharmaceutical formulation comprising a compound of formula (I) as claimed in any one of claims 1 to 3, or a pharmaceutically-acceptable salt thereof, associated with a pharmaceutically-acceptable carrier therefor.

6. A pharmaceutical formulation as claimed in claim 5, wherein the compound of formula (I) has the structure:

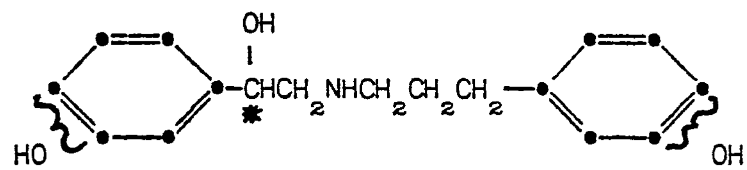

or a pharmaceutically-acceptable salt thereof.

7. A process for preparing a compound of formula (I) as claimed in any one of claims 1 to 3, or a pharmaceutically acceptable salt thereof; which process comprises:

(a) removing the protecting group Q from a protected amine of formula (II):

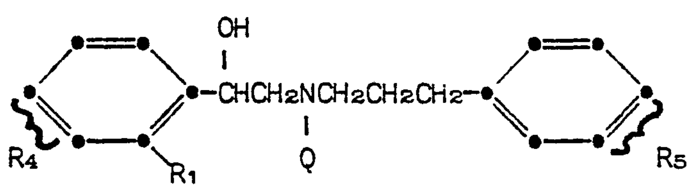

(II)

where $R_1$ is as defined in claim 1, $R_4$ and $R_5$ are respectively $R_2$ and $R_3$ as defined in claim 1 or independently can represent -OQ; or

(b) reducing a ketone of formula (III):

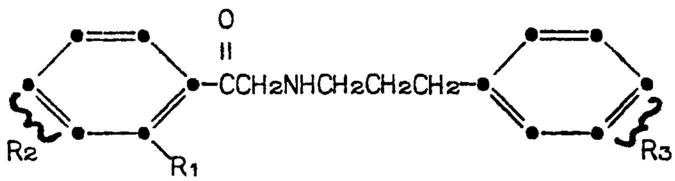

(III)

where $R_1$, $R_2$ and $R_3$ are as defined in claim 1, and

(c) where appropriate, resolving racemic mixtures of non-optically active products into the optically active R-isomer.

8. A compound of formula (I), or a pharmaceutically-acceptable salt thereof, whenever prepared by a process according to claim 7.

9. A protected amine of formula (II) or a ketone of formula (III) as defined in claim 7.

10. A compound of formula (I) as claimed in any one of claims 1 to 4, for use as an inotropic agent.

European Patent Office

# EUROPEAN SEARCH REPORT

Application number
EP 79 301 281.6

| DOCUMENTS CONSIDERED TO BE RELEVANT | | | CLASSIFICATION OF THE APPLICATION (Int. Cl.) |
|---|---|---|---|
| Category | Citation of document with indication, where appropriate, of relevant passages | Relevant to claim | |
| A | FR - M - 7 198 (N.V. PHILIPS)<br>-- | | C 07 C 91/30<br>A 61 K 31/135<br>C 07 C 91/34 |
| A | Chemical Abstracts, Volume 80, Nr. 17, April 29, 1974<br>(COLUMBUS, OHIO, USA)<br>J. Van DIJK et al. "Synthesis of ß-phenylethylamine derivatives. X.N-(Hydroxy- and methoxyaralkyl) derivatives", page 366, column 2, abstract Nr. 95398 Z<br>& Recl. Trav. Chim., Pays-Bas, Volume 92, Nr. 12, 1973, pages 1281 to 1297 (Eng)<br>---- | | TECHNICAL FIELDS SEARCHED (Int.Cl.)<br>A 61 K 31/135<br>C 07 C 91/30<br>C 07 C 91/34 |

CATEGORY OF CITED DOCUMENTS
X: particularly relevant
A: technological background
O: non-written disclosure
P: intermediate document
T: theory or principle underlying the invention
E: conflicting application
D: document cited in the application
L: citation for other reasons
&: member of the same patent family, corresponding document

X The present search report has been drawn up for all claims

| Place of search | Date of completion of the search | Examiner |
|---|---|---|
| Berlin | 18-10-1979 | KAPTEYN |